# EUROPÄISCHE PATENTSCHRIFT

(11) **EP 2 061 868 B1**
(45) Veröffentlichungstag und Bekanntmachung des Hinweises auf die Patenterteilung: **19.01.2011**
(21) Anmeldenummer: 07818140.1
(22) Anmeldetag: 13.09.2007
(51) Int. Cl.: C12M 1/107

(54) **GÄRBEHÄLTER SOWIE BIOGASANLAGE MIT EINEM GÄRBEHÄLTER**
FERMENTATION CONTAINER AND BIOGAS INSTALLATION COMPRISING A FERMENTATION CONTAINER
RÉCIPIENT DE FERMENTATION ET INSTALLATION À BIOGAZ COMPRENANT UN RÉCIPIENT DE FERMENTATION

(30) Priorität: 15.09.2006 DE 202006014149 U
(43) Veröffentlichungstag der Anmeldung: 27.05.2009
(73) Patentinhaber: Reinelt, Gerhard, 49124 Georgsmarienhütte (DE)
(72) Erfinder: Reinelt, Gerhard, 49124 Georgsmarienhütte (DE)
(74) Vertreter: Pott, Ulrich
(86) Internationale Anmeldenummer: PCT/EP2007/007976
(87) Internationale Veröffentlichungsnummer: WO 2008/031591

(56) Entgegenhaltungen:
- WO-A-97/33705
- DE-A1- 4 118 415
- DE-A1- 10 042 976
- DE-C1- 19 958 491
- DE-U1-202006 014 148

## Beschreibung

Die Erfindung betrifft einen Gärbehälter nach dem Oberbegriff des Anspruchs 1 sowie eine Biogasanlage zur Erzeugung von Biogas, insbesondere Methan, mit einem derartigen Gärbehälter.

Vorgenannte Gärbehälter sowie Biogasanlagen sind im Stand der Technik bekannt. Dabei wird der Gärraum umseitig von einer Betonwanne oder einer massiven Stahlwanne begrenzt, die jeweils mit einer flüssigkeitsundurchlässigen Membran ausgelegt sind. Die Kosten derartiger Gärbehälter und Biogasanlagen sind jedoch relativ hoch. Das Gießen der Betonwanne erfolgt arbeitsintensiv direkt am Aufstellungsort des Gärbehälters. Die massive Stahlwanne ist materialbedingt teuer und muß ebenfalls arbeitsintensiv am Aufstellungsort aus Einzelelementen zusammengeschweißt werden.

Der Erfindung liegt die Aufgabe zugrunde, einen kostengünstigen Gärbehälter zu entwickeln sowie eine Biogasanlage mit einem derartigen Gärbehälter bereitzustellen.

Die Erfindung löst diese Aufgabe durch einen Gärbehälter mit den Merkmalen des Anspruchs 1 sowie durch eine Biogasanlage mit den Merkmalen des Anspruchs 11. Vorteilhafte Ausgestaltungen der Erfindung ergeben sich aus den abhängigen Ansprüchen 2 bis 10 und 12 bis 14.

Dadurch, daß die Membran außenseitig von einem Gitternetz stabilisiert wird, wobei das Gitternetz mit einem Stützelement verbunden ist, das das Gitternetz hält, ist die Konstruktion der Begrenzung des Gärraumes sehr kostengünstig. Die überwiegende Anzahl der Teile des Gärbehälters oder auch der Biogasanlage können bereits vorab gefertigt werden und müssen vor Ort lediglich montiert werden. Hierdurch können deutlich Personalkosten eingespart werden. Aufgrund der Leichtbaukonstruktion und des daraus bedingten geringen Gewichtes des Gärbehälters sind dessen Teile leichter zu transportieren und zudem wird auch lediglich ein weniger dickes Fundament mit einer ggf. auch weniger aufwendigen Verankerung im Boden benötigt, so daß auch hier Kosten eingespart werden können.

Das Gitternetz wird in einer vorteilhaften Ausgestaltung von einem Kunststoffnetz oder einem Stahldrahtnetz, also einem Netz aus Kunststoff oder Stahldraht, gebildet. Diese sind leicht zu verarbeiten und zudem preiswert, so daß sich die Kosten des Gärbehälters durch diese Ausgestaltung weiter reduzieren lassen. Das Kunststoffnetz hat gegenüber dem Stahldrahtnetz den weitergehenden Vorteil, noch einmal preisgünstiger und leichter bei gleicher Festigkeit zu sein, so daß insbesondere bei Verwendung eines Kunststoffnetzes deutlich Kosten eingespart werden können, ohne daß die Funktionalität des Gitternetzes eingeschränkt ist.

Der Gärbehälter hat in einer bevorzugten Ausgestaltung der Erfindung ein Heizsystem, mit dessen Hilfe die Fermentationslösung auf eine gewünschte Temperatur gebracht und dort gehalten werden kann, um den Fermentationsprozeß zu fördern. Das Heißsystem weist bevorzugt eine weitere Membran auf, die parallel zur ersten Membran angeordnet ist und mit dieser einen Wasserleitungsraum bildet. Dabei strömt warmes Wasser durch einen Eingangsstutzen zwischen die erste Membran und die weitere Membran in den Wasserleitungsraum und erwärmt währenddessen die Fermentationslösung, bevor das Wasser aus einem Ausgangsstutzen wieder herausströmt. Der so gebildete Wasserleitungsraum kann den Gärraum vollflächig umschließen oder diesen auch in Bahnen spiralförmig umgeben. Hierdurch ist eine einfache und kostengünstige Temperierung der Fermentationslösung gewährleistet. Alternativ oder ergänzend kann im Bodenbereich des Gärraumes eine Bodenheizung vorgesehen sein.

Weitere Einzelheiten und Vorteile der Erfindung ergeben sich aus den Unteransprüchen sowie den nachfolgend beschriebenen schematischen Ausführungsbeispielen; es zeigen:
- Fig. 1: einen erfindungsgemäßen Gärbehälter in der Draufsicht,
- Fig. 2: eine Schnittdarstellung des Gegenstandes aus Fig. 1 nach der Linie A-A in Fig. 1,
- Fig. 3: eine Ausschnittsvergrößerung des Details B aus Fig. 2,
- Fig. 4: eine Ausschnittsvergrößerung des Details C aus Fig. 2,
- Fig. 5: eine erfindungsgemäße Biogasanlage in der Draufsicht,
- Fig. 6: die Biogasanlage aus Fig. 5 in einer Schnittdarstellung nach der Linie A-A in Fig. 1 und
- Fig. 7: eine Ausschnittsvergrößerung des Details D aus Fig. 6.

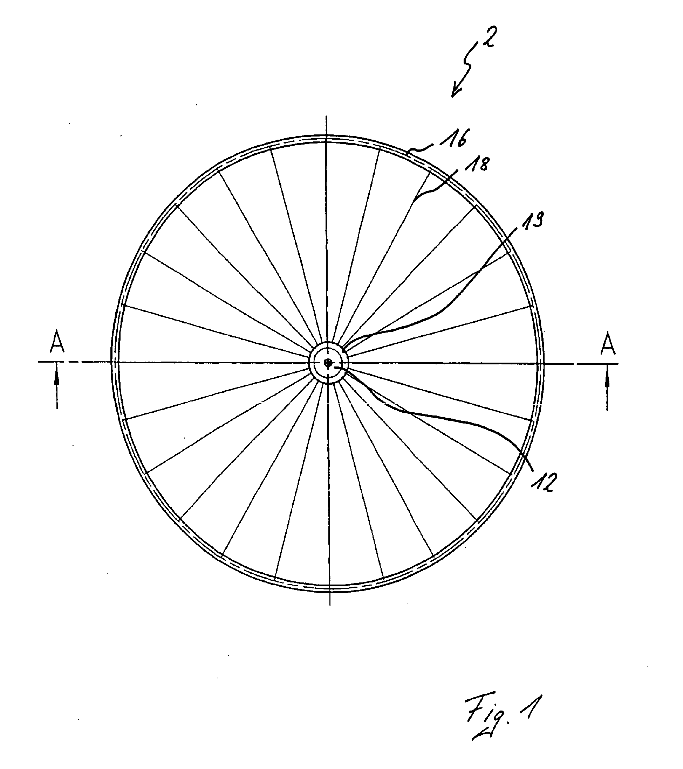

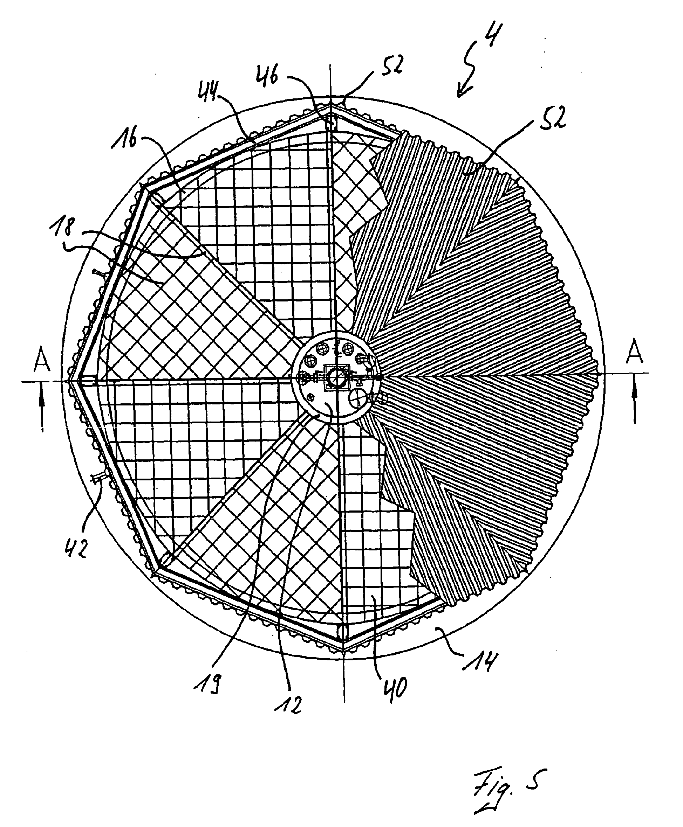

Nachfolgend werden gleichwirkende Teile mit einem einheitlichen Bezugszeichen versehen. Die Figuren 1 bis 4 zeigen insgesamt einen erfindungsgemäßen Gärbehälter 2 in verschiedenen Ansichten bzw. Ausschnitten. Fig. 5 bis Fig. 7 zeigen dagegen eine erfindungsgemäße Biogasanlage 4 mit einem erfindungsgemäßen Gärbehälter 2 ebenfalls in verschiedenen Ansichten bzw. Ausschnitten.

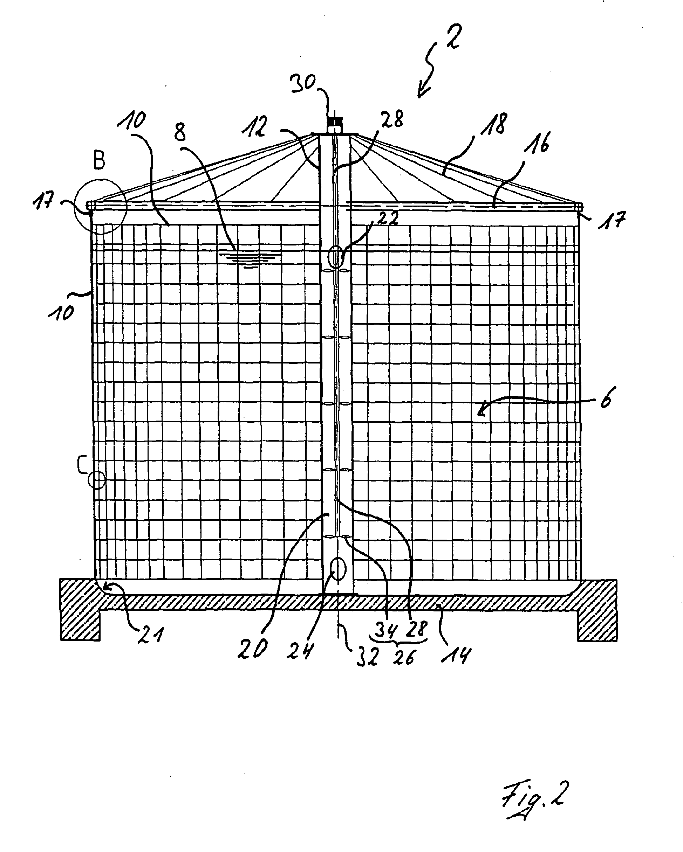

Fig. 1 zeigt den Gärbehälter 2 in der Draufsicht, während Fig. 2 den Gärbehälter in einer Schnittdarstellung nach der in Fig. 1 dargestellten Linie A-A mit einem zum Betrachter hin offenen Gärraum 6. Der Gärraum 6 wird umseitig von einer flüssigkeitsundurchlässigen Membran 8 begrenzt, die in Fig. 2 durch parallele, ein Dreieck andeutende Linien illustriert ist. Die Membran 8 wird außenseitig bezogen auf den Gärraum 6 von einem Gitternetz 10 umgeben und durch dieses stabilisiert.

Das Gitternetz 10 ist über eine Trageinrichtung mit einem Stützelement 12 verbunden und wird von dem Stützelement 12 gehalten. Das Stützelement 12 ist auf einem Fundament 14 abgestützt und befestigt und im wesentlichen lotrecht ausgerichtet. Die Trageinrichtung hat einen Tragring 16, der über Abspannungen 18 von dem Stützelement 12 getragen wird und an dem das Gitternetz 10 sowie im vorliegenden Fall auch die Membran 8 befestigt sind.

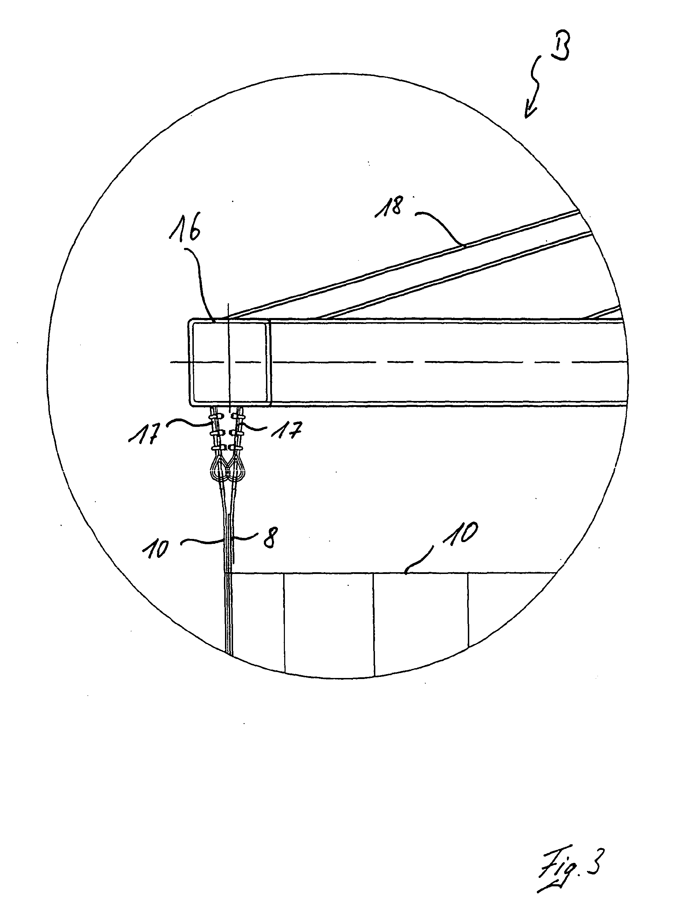

Die Befestigung ist in einer Ausschnittsvergrößerung des Ausschnittes B aus Fig. 2 in Fig. 3 veranschaulicht. Am Tragring 16 sind Halterungen 17 befestigt, in die zum einen das Gitternetz 10 und zum anderen die Membran 8 eingehängt sind. In Fig. 2 sind lediglich vier derartige Halterungen 17 dargestellt. Bevorzugt sind jedoch eine Vielzahl Halterungen 17 gleichmäßig verteilt am Tragring 16 befestigt.

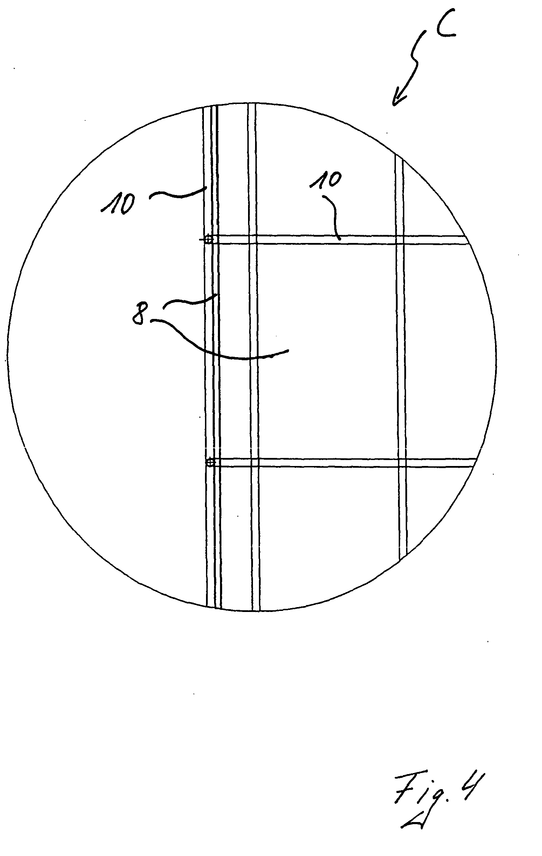

Fig. 4 zeigt den Aufbau der Begrenzung des Gärraumes 6 im Detail. Die Membran 8 ist außenseitig von dem Gitternetz 10 umgeben und wird dadurch stabilisiert.

Wie aus Fig. 1 und Fig. 2 ersichtlich, weist der Gärbehälter 2 lediglich ein Stützelement 12 auf, das in der Mitte des Gärraumes 6 angeordnet ist. Das Stützelement 12 wird dabei von dem Tragring 16 umgeben. Die an dem Tragring 12 befestigte Membran 8 sowie das diese umgebende Gitternetz 10 hängen an dem Tragring 16 herab und bilden durch dessen kreisförmige Ausgestaltung eine im wesentlichen zylindrische Form des Gärraumes 6. Durch die Wahl der Form des Tragringes 16 (z.B. kreisrund, oval oder auch eckig) kann in einfacher Weise die Querschnittsform des Gärraumes 6 variiert werden. Durch die Ausgestaltung des Gärbehälters 2 mit lediglich einem in der Mitte des Gärraumes 6 angeordneten Stützelement 12 ist zudem eine besonders kostengünstige Ausgestaltung des Gärbehälters 6 möglich.

Die Abspannungen 18 können von Seilen, Ketten, Netzen, Streben oder dergleichen Verbindungselemente zwischen Stützelement 12 und Tragring 16 allein oder in Kombination gebildet werden. Die Abspannungen 18 haben dabei bevorzugt einen gleichbleibenden Abstand zueinander, um das Gewicht des Tragringes 16 sowie des Gitternetzes 10 und der Membran 8 gleichmäßig auf das Stützelement 12 zu übertragen. Das Stützelement weist auf der dem Fundament 14 abgewandten Seite eine der Form des Tragringes 16 angepaßte Platte 19 auf, an der die Abspannungen befestigt sind. Hierdurch ist eine gute Einleitung der Gewichtskräfte des Tragringes 16 und der daran angeordneten Teile 8, 10 in das Stützelement 12 gewährleistet.

Das Gitternetz 10 ist bevorzugt am Fundament 14 fixiert. Dadurch kann ein Teil der auf das Gitternetz 10 einwirkenden Kräfte im gefüllten Zustand des Gärbehälters 2 auf das Fundament 14 abgeleitet werden. Das Stützelement 12 wird durch diese Ausgestaltung deutlich entlastet. Mit Vorteil ist zudem der Übergang der bodenseitigen Begrenzung des Gärraumes 6 zur seitlichen, vertikalen Begrenzung des Gärraumes 6 mit einer Abrundung 21 versehen. Durch diese abgerundete Ausbildung ist der von der Fermentationslösung auf die Begrenzung im Bereich der Abrundung 21 einwirkende Druck deutlich niedriger als bei einem eckig ausgebildeten Übergang. Der Gärraum 6 wird von dem Fundament 14 begrenzt. Die Membran 8 ist dabei flüssigkeitsdicht mit dem Fundament 14 verbunden. Der Gärraum 6 kann jedoch auch unterseitig von der Membran 8 begrenzt werden, die dann auf dem Fundament 14 aufliegt (hier nicht dargestellt).

Wie aus Fig. 2 ersichtlich, weist das Stützelement 12 einen innenliegenden Hohlraum 20 auf, der über eine erste Öffnung 22 und eine weitere Öffnung 24 mit dem Gärraum 6 verbunden ist. Dabei ist das Stützelement 12 bevorzugt wie dargestellt als Rohr ausgebildet. In dem Hohlraum 20 ist eine Fördereinrichtung 26 zur Beförderung von durch die erste Öffnung 22 in den Hohlraum 20 hineinströmender Fermentationsflüssigkeit hin zur weiteren Öffnung 24 und durch diese hindurch wieder in den Gärraum 6. Hierdurch kann in einfacher Weise eine Umwälzung der Fermentationsflüssigkeit in dem Gärraum 6 erreicht werden. Die Fördereinrichtung 26 weist eine Antriebswelle 28 auf, die von einem Motor 30 angetrieben wird und an der quer zur Längsachse 32 rotierende Schaufelräder 34, ein Propeller oder eine Schraube angeordnet sind.

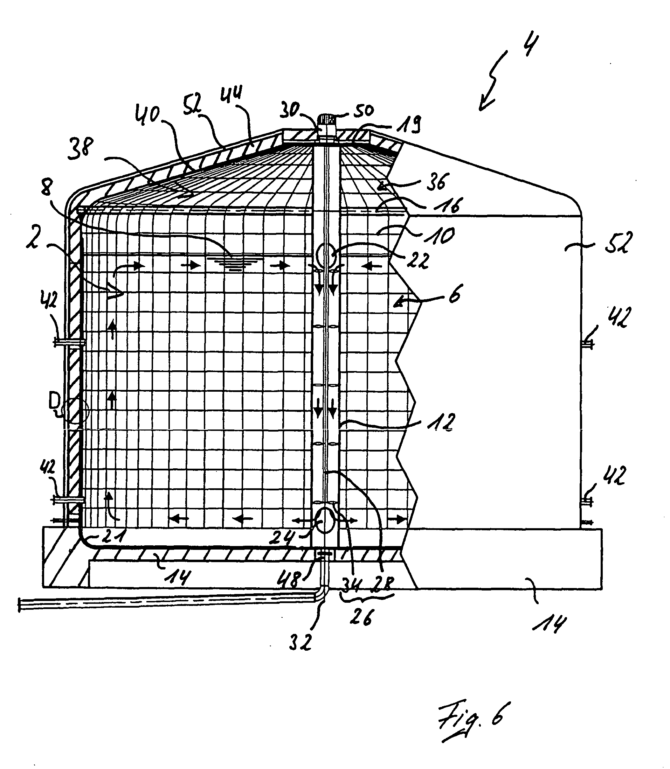

Eine erfindungsgemäße Biogasanlage 4 ist in Fig. 5 in der Draufsicht sowie in Fig. 6 in einer teilweise geschnittenen Darstellung dargestellt. Die Biogasanlage 4 weist einen Gärbehälter 2 sowie einen oberhalb des Gärbehälters 2 angeordneten Gasspeicher 36 auf. Der Gasspeicher 36 ist dabei gasdicht mit dem Gärbehälter 2 verbunden. In dem Gärbehälter 2 befindet sich eine Fermentationslösung, wie z.B. Gülle oder Jauche, aus der über Fermentationsprozesse Biogas, insbesondere Methan, gebildet wird. Das Biogas wird dann im Gasspeicher 36 bevorzugt unter Druck gespeichert.

Der Gasspeicher 36 weist einen Gasraum 38 auf, der bereichsweise von einer gasdichten Wandung 40 begrenzt ist. Die Wandung 40 ist dabei unterhalb der Abspannungen 18 des Tragringes 16 angeordnet und wird in einer konstruktiv einfachen Ausgestaltung von einer flexiblen, gasdichten Folie 40 gebildet. Die Abspannungen 18 werden im Ausführungsbeispiel von Netzen und von Streben gemeinsam gebildet.

Das Stützelement 12 ist auch in dem Ausführungsbeispiel der Fig. 5 bis Fig. 7 hohlgeformt und weist eine erste Öffnung 22 sowie eine weitere Öffnung 24 und eine Fördereinrichtung 26 auf. Die Umwälzung der Fermentationsflüssigkeit durch die Fördereinrichtung 26 ist in Fig. 6 durch Pfeile veranschaulicht.

Der Gärbehälter 2 weist ein Heizsystem 42 auf, mit dessen Hilfe die Fermentationslösung auf eine gewünschte Temperatur gebracht und dort gehalten werden kann, um den Fermentationsprozeß zu fördern. Der Gärbehälter 2 und der Gasspeicher 36 sind zudem von einer Isolierung 44 umgeben, um die gewünschte Temperatur energiesparender halten zu können. Die Isolierung 44 kann dabei ebenfalls von dem Stützelement 12 getragen werden oder aber durch vertikale Profile 46, die auf dem Fundament 14 verankert und gleichmäßig um den Gärbehälter 2 herum verteilt sind.

Der Gärbehälter 2 hat im Bodenbereich des Gärraumes 6 einen Ein- und Auslaß 48 zur Befüllung bzw. Entleerung des Gärbehälters 2 mit Fermentationsflüssigkeit. Zur Entnahme von Gas aus dem Gasspeicher 36 ist die Biogasanlage 4 mit einem Gasausgang 50 ausgestattet.

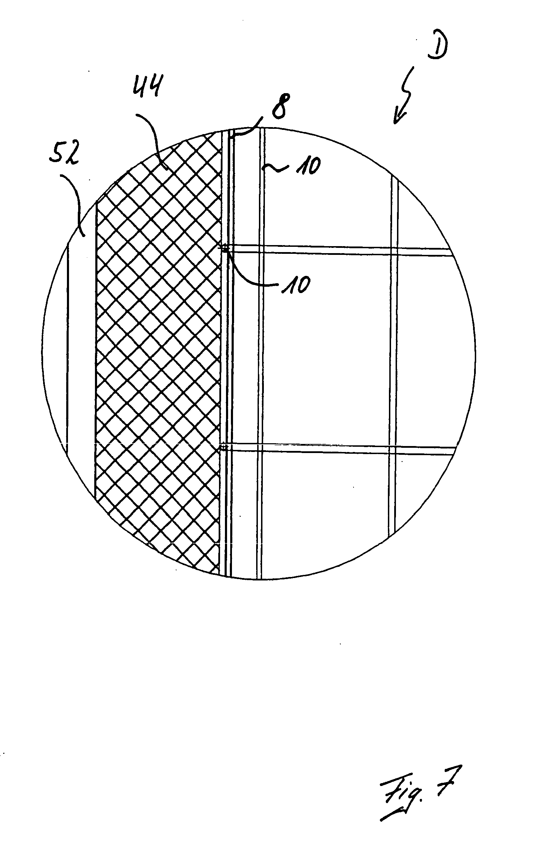

Fig. 7 zeigt den Aufbau der Begrenzung des Gärraumes 6 im Detail. Die Membran 8 wird nach außen hin durch das Gitternetz 10 stabilisiert. Membran 8 und Gitternetz 10 werden außerhalb des Gärraumes 6 von der Isolierung 44 umschlossen. Zur mechanischen Absicherung der Isolierung 44 ist diese abschließend mit einer Verkleidung 52 versehen.

## Patentansprüche

1. Biogasanlage zur Erzeugung von Biogas, insbesondere Methan (4), mit einem Gärbehälter (2) und einem Gasspeicher (36), der gasdicht mit dem Gärbehälter (2) verbunden ist, wobei der Gärbehälter (2) einen Gärraum (6) zur Aufnahme von Gülle, Jauche oder dergleichen Fermentationsflüssigkeit aufweist und der Gärraum (6) umseitig zumindest bereichsweise von einer flüssigkeitsundurchlässigen Membran (8) begrenzt ist, **gekennzeichnet durch** wenigstens ein Gitternetz (10),
das die Membran (8) außenseitig stabilisiert und **durch** zumindest ein Stützelement (12) das mit dem Gitternetz (10) dieses Halten verbunden ist.

2. Gärbehälter (2) nach Anspruch 1, **dadurch gekennzeichnet, daß** das Stützelement (12) auf oder in einem Fundament (14) des Gärbehälters (2) abgestützt ist.

3. Gärbehälter (2) nach einem der vorhergehenden Ansprüche, **dadurch gekennzeichnet, daß** das Stützelement (12) die Membran (8) haltend mit dieser verbunden ist.

4. Gärbehälter (2) nach einem der vorhergehenden Ansprüche, **dadurch gekennzeichnet, daß** der Gärbehälter (2) lediglich ein Stützelement (12) aufweist, das in der Mitte des Gärraumes (6) angeordnet ist.

5. Gärbehälter (2) nach einem der vorhergehenden Ansprüche, **dadurch gekennzeichnet, daß** an dem Stützelement (12) eine Trageinrichtung (16, 18) fixiert ist, an der das Gitternetz (10) und /oder die Membran (8) befestigt sind.

6. Gärbehälter (2) nach Anspruch 5, **dadurch gekennzeichnet, daß** die Trageinrichtung (16, 18) einen Tragring (16) aufweist, der über Abspannungen (18) von dem Stützelement (12) getragen wird und an dem das Gitternetz (10) und /oder die Membran (8) befestigt sind.

7. Gärbehälter (2) nach einem der Ansprüche 2 bis 6, **dadurch gekennzeichnet, daß** das Gitternetz (10) und/oder die Membran (8) mit dem Fundament (14) verbunden sind.

8. Gärbehälter (2) nach einem der vorhergehenden Ansprüche, **dadurch gekennzeichnet, daß** das Stützelement (12) einen innenliegenden Hohlraum (20) aufweist, der über wenigstens eine erste (22) und eine weitere Öffnung (24) mit dem Gärraum (6) verbunden ist, wobei in dem Hohlraum (20) eine Fördereinrichtung (26) zur Beförderung von durch die erste Öffnung (22) in den Hohlraum (20) eindringender Fermentationsflüssigkeit zur weiteren Öffnung (24) hin angeordnet ist.

9. Gärbehälter (2) nach einem der vorhergehenden Ansprüche, **gekennzeichnet durch**, einen Ein- und Auslaß (48) für die Fermentationslösung im Bodenbereich des Gärraumes (6).

10. Gärbehälter (2) nach einem der vorhergehenden Ansprüche, **dadurch gekennzeichnet, daß** das Gitternetz (10) von einem Kunststoffnetz gebildet ist.

11. Biogasanlage zur Erzeugung von Biogas, insbesondere Methan, (4) mit einem Gärbehälter (2) nach einem der vorhergehenden Ansprüche und einem Gasspeicher (36), der gasdicht mit dem Gärbehälter (2) verbunden ist.

12. Biogasanlage (4) nach Anspruch 11, **dadurch gekennzeichnet, daß** der Gasspeicher (36) oberhalb des Gärbehälters (2) angeordnet ist.

13. Biogasanlage (4) nach einem der Ansprüche 11 oder 12, **dadurch gekennzeichnet, daß** der Gasspeicher (36) einen Gasraum (38) aufweist, der zumindest bereichsweise von einer gasdichten Wandung (40) begrenzt ist.

14. Biogasanlage (4) nach Anspruch 13 und 6, **dadurch gekennzeichnet, daß** die Wandung (40) unterhalb der Abspannungen (18) des Tragringes (16) angeordnet ist.

## Claims

1. A biogas plant for the generation of biogas, in particular methane (4), with a fermentation container (2) and a gas storage unit (36), which is connected in a gas-tight manner to the fermentation container (2), wherein the fermentation container (2) comprises a fermentation chamber (6) for receiving liquid manure, sewage or a similar fermentation liquid and at least part of the periphery of the fermentation chamber (6) is delimited by a membrane (8) that is impermeable to liquid, **characterised by** at least one grid structure (10), which stabilises the exterior of membrane (8) and by at least one support element (12) which is connected to the grid structure (10) holding the latter.

2. The fermentation container (2) according to claim 1, **characterised in that** the support element (12) is supported on or in a base (14) of the fermentation container (2).

3. The fermentation container (2) according to any one of the preceding claims, **characterised in that** the support element (12) is connected to the membrane (8) holding the latter.

4. The fermentation container (2) according to any one of the preceding claims, **characterised in that** the fermentation container (2) comprises only one support element (12), which is disposed in the middle of the fermentation chamber (6).

5. The fermentation container (2) according to any one of the preceding claims, **characterised in that** there is fixed on the support element (12) a carrying arrangement (16, 18), to which the grid structure (10) and/or the membrane (8) are fastened.

6. The fermentation container (2) according to claim 5, **characterised in that** the carrying arrangement (16, 18) comprises a carrying ring (16), which is carried via bracing elements (18) by the support element (12) and is fastened to the grid structure (10) and/or the membrane (8).

7. The fermentation container (2) according to any one of claims 2 to 6, **characterised in that** the grid structure (10) and/or the membrane (8) are connected to the base (14).

8. The fermentation container (2) according to any one of the preceding claims, **characterised in that** the support element (12) comprises an inner hollow space (20), which is connected to the fermentation chamber (6) via at least a first (22) and a further opening (24), there being disposed in the hollow space (20) a conveying device (26) for conveying fermentation liquid penetrating through the first opening (22) into the hollow space (20) to further opening (24).

9. The fermentation container (2) according to any one of the preceding claims, **characterised by** an inlet and an outlet (48) for the fermentation solution in the bottom region of the fermentation chamber (6).

10. The fermentation container (2) according to any one of the preceding claims, **characterised in that** the grid structure (10) is formed by a plastic structure.

11. A biogas plant for the generation of biogas, in particular methane (4), with a fermentation container (2) according to any one of the preceding claims and a gas storage unit (36), which is connected in a gas-tight manner to the fermentation container (2).

12. The biogas plant (4) according to claim 11, **characterised in that** the gas storage unit (36) is disposed above the fermentation container (2).

13. The biogas plant (4) according to any one of claims 11 or 12, **characterised in that** the gas storage unit (36) comprises a gas chamber (38), which is bounded at least in part by a gas-tight wall (40).

14. The biogas plant (4) according to claim 13 and 6, **characterised in that** the wall (40) is disposed beneath the bracing elements (18) of the carrying ring (16).

## Revendications

1. Installation de production de biogaz, en particulier de méthane (4), comprenant un récipient de fermentation (2) et un réservoir de gaz (36) qui est relié au récipient de fermentation (2) de manière étanche aux gaz, lequel récipient de fermentation (2) présente une enceinte de fermentation (6), destinée à recevoir du lisier, du purin ou un liquide à fermenter similaire, et laquelle enceinte de fermentation (6) est délimitée au dos, du moins par zones, par une membrane imperméable aux liquides (8), **caractérisée par** au moins un grillage (10) qui stabilise la membrane (8) à l'extérieur et par au moins un élément d'appui (12) qui est relié au grillage (10) de façon à le maintenir.

2. Récipient de fermentation (2) selon la revendication 1, **caractérisé en ce que** l'élément d'appui (12) est supporté sur ou dans une plaque de fondation (14) du récipient de fermentation (2).

3. Récipient de fermentation (2) selon l'une des revendications précédentes, **caractérisé en ce que** l'élément d'appui (12) est relié à la membrane (8) de manière à la maintenir.

4. Récipient de fermentation (2) selon l'une des revendications précédentes, **caractérisé en ce que** le récipient de fermentation (2) présente un seul élément d'appui (12) qui est disposé au centre de l'enceinte de fermentation (6).

5. Récipient de fermentation (2) selon l'une des revendications précédentes, **caractérisé en ce qu'**un dispositif support (16, 18) auquel le grillage (10) et/ou la membrane (8) sont fixés est monté sur l'élément d'appui (12).

6. Récipient de fermentation (2) selon la revendication 5, **caractérisé en ce que** le dispositif support (16, 18) présente un anneau porteur (16) qui est supporté par l'élément d'appui (12) au moyen de haubans (18) et auquel le grillage (10) et/ou la membrane (8) sont fixés.

7. Récipient de fermentation (2) selon l'une des revendications 2 à 6, **caractérisé en ce que** le grillage (10) et/ou la membrane (8) sont reliés à la plaque de fondation (14).

8. Récipient de fermentation (2) selon l'une des revendications précédentes, **caractérisé en ce que** l'élément d'appui (12) présente une cavité intérieure (20) qui est reliée par au moins un premier orifice (22) et un autre orifice (24) à l'enceinte de fermentation (6), un dispositif transporteur (26) étant disposé dans la cavité (20) afin de transporter le liquide à fermenter qui pénètre dans la cavité (20) par le premier orifice (22) en direction de l'autre orifice (24).

9. Récipient de fermentation (2) selon l'une des revendications précédentes, **caractérisé par** une entrée et une sortie (48) pour la solution fermentée dans la zone du fond de l'enceinte de fermentation (6).

10. Récipient de fermentation (2) selon l'une des revendications précédentes, **caractérisé en ce que** le grillage (10) est constituée d'une grille en matière plastique.

11. Installation de production de biogaz, en particulier de méthane (4), comprenant un récipient de fermentation (2) selon l'une des revendications précédentes et un réservoir de gaz (36) qui est relié au récipient de fermentation (2) de manière étanche aux gaz.

12. Installation de production de biogaz (4) selon la revendication 11, **caractérisée en ce que** le réservoir de gaz (36) est disposé au-dessus du récipient de fermentation (2).

13. Installation de production de biogaz (4) selon l'une des revendications 11 ou 12, **caractérisée en ce que** le réservoir de gaz (36) présente un espace gazeux (38) qui est délimité au moins par zones par une paroi étanche aux gaz (40).

14. Installation de production de biogaz (4) selon la revendication 13 et la revendication 6, **caractérisé en ce que** la paroi (40) est disposée en dessous des haubans 18) de l'anneau porteur (16).
